# EUROPEAN PATENT SPECIFICATION

(11) **EP 3 801 564 B1**
(45) Date of publication and mention of the grant of the patent: **24.06.2026**
(21) Application number: 19732481.7
(22) Date of filing: 04.06.2019
(51) Int. Cl.: A61K 31/765, A61K 33/10, A61K 33/14, A61P 1/10

(54) **PHARMACEUTICAL COMPOSITION FOR TREATING CHRONIC CONSTIPATION**
PHARMAZEUTISCHE ZUSAMMENSETZUNG ZUR BEHANDLUNG VON CHRONISCHER VERSTOPFUNG
COMPOSITION PHARMACEUTIQUE POUR LE TRAITEMENT DE LA CONSTIPATION CHRONIQUE

(30) Priority: 11.06.2018 JP 2018110910; 12.09.2018 JP 2018170377
(43) Date of publication of application: 14.04.2021
(73) Proprietor: Norgine BV, 1083 HP Amsterdam (NL)
(72) Inventor: SHINBO, Kazuhiko, Tokyo 104-0042 (JP); FUJITA, Shinichi, Tokyo 104-0042 (JP); FUKUDA, Emi, Tokyo 104-0042 (JP); SHIMADA, Misae, Tokyo 104-0053 (JP); NONOSHITA, Masaru, Tokyo 104-0053 (JP)
(74) Representative: Abel & Imray LLP
(86) International application number: PCT/JP2019/022180
(87) International publication number: WO 2019/239963

(56) References cited:
- CINCA R ET AL: "Randomised clinical trial: macrogol/PEG 3350+electrolytes versus prucalopride in the treatment of chronic constipation - a comparison in a controlled environment", ALIMENTARY PHARMACOLOGY & THERAPEUTICS, BLACKWELL SCIENTIFIC PUBLICATIONS LTD., CAMBRIDGE, GB, vol. 37, no. 9, 11 March 2013 (2013-03-11), pages 876 - 886, XP071542416, ISSN: 0269-2813, DOI: 10.1111/APT.12278
- CORAZZIARI E ET AL: "LONG TERM EFFICACY, SAFETY, AND TOLERABILITY OF LOW DAILY DOSES OF ISOSMOTIC POLYETHYLENE GLYCOL ELECTROLYTE BALANCED SOLUTION (PMF-100) IN THE TREATMENT OF FUNCTIONAL CHRONIC CONSTIPATION", GUT, BRITISH MEDICAL ASSOCIATION, LONDON, UK, vol. 46, no. 4, 1 April 2000 (2000-04-01), pages 522 - 526, XP009060191, ISSN: 0017-5749, DOI: 10.1136/GUT.46.4.522
- J. A. DI PALMA ET AL: "An open-label study of chronic polyethylene glycol laxative use in chronic constipation : PEG FOR TREATMENT OF CHRONIC CONSTIPATION", ALIMENTARY PHARMACOLOGY & THERAPEUTICS., vol. 25, no. 6, 22 January 2007 (2007-01-22), GB, pages 703 - 708, XP055619288, ISSN: 0269-2813, DOI: 10.1111/j.1365-2036.2006.03228.x
- SUN HWAN BAE: "Long-term safety of PEG 4000 in children with chronic functional constipation: A biochemical perspective", KOREAN JOURNAL OF PEDIATRICS, vol. 53, no. 7, 1 January 2010 (2010-01-01), pages 741, XP055619256, ISSN: 1738-1061, DOI: 10.3345/kjp.2010.53.7.741
- CHASSAGNE PHILIPPE ET AL: "Tolerance and long-term efficacy of polyethylene glycol 4000 (Forlax ) compared to lactulose in elderly patients with chronic constipation", JOURNAL OF NUTRITION, HEALTH AND AGING, SPRINGER PARIS, PARIS, vol. 21, no. 4, 22 June 2016 (2016-06-22), pages 429 - 439, XP036203144, ISSN: 1279-7707, [retrieved on 20160622], DOI: 10.1007/S12603-016-0762-6
- BADIALI D ET AL: "USE OF LOW DOSE POLYETHYLENE GLYCOL SOLUTIONS IN THE TREATMENT OF FUNCTIONAL CONSTIPATION", ITALIAN JOURNAL OF GASTROENTEROLOGY AND HEPATOLOGY, PACINI EDITORE, PISA, IT, vol. 31, no. SUPPL. 03, 1 January 1999 (1999-01-01), pages S245 - S248, XP009060183, ISSN: 1125-8055
- ERICKSON ET AL: "Polyethylene Glycol 3350 for Constipation in Children With Dysfunctional Elimination", JOURNAL OF UROLOGY, LIPPINCOTT WILLIAMS & WILKINS, BALTIMORE, MD, US, vol. 170, no. 4, 1 October 2003 (2003-10-01), pages 1518 - 1520, XP005533081, ISSN: 0022-5347, DOI: 10.1097/01.JU.0000083730.70185.75
- PATRICIA BOECHAT GOMES ET AL: "Comparison of the effectiveness of polyethylene glycol 4000 without electrolytes and magnesium hydroxide in the treatment of chronic functional constipation in children", JORNAL DE PEDIATRIA, vol. 0, no. 0, 29 November 2010 (2010-11-29), BR, XP055619275, ISSN: 0021-7557, DOI: 10.2223/JPED.2051
- SONIA MICHAIL ET AL: "Downloaded from https: Polyethylene Glycol for Constipation in Children Younger Than Eighteen Months Old", 1 August 2004 (2004-08-01), XP055619269, Retrieved from the Internet <URL:https://journals.lww.com/jpgn/Fulltext/2004/08000/Polyethylene_Glycol_for_Constipation_in_Children.14.aspx#pdf-link> [retrieved on 20190906]
- I. MIGEON-DUBALLET ET AL: "Long-term efficacy and cost-effectiveness of polyethylene glycol 3350 plus electrolytes in chronic constipation: a retrospective study in a disabled population", CURRENT MEDICAL RESEARCH AND OPINION, vol. 22, no. 6, 21 June 2006 (2006-06-21), GB, pages 1227 - 1235, XP055619340, ISSN: 0300-7995, DOI: 10.1185/030079906X112543
- NORGINE TEAM: "Movicol aromafrei", 1 April 2011 (2011-04-01), XP055619483, Retrieved from the Internet <URL:https://www.google.de/url?sa=t&rct=j&q=&esrc=s&source=web&cd=2&ved=2ahUKEwjU95iUlMPkAhWGsRQKHRZGB9oQFjABegQIChAE&url=https%3A%2F%2Fwww.movicol.de%2Ffileadmin%2Fuser_upload%2Fgebrauchsinformationen%2Fgebrauchsinfo_movicol_aromafrei.pdf&usg=AOvVaw3rGc5Xl8Jn2u-ABVuaVc9m> [retrieved on 20190906]
- NORGINE B.V.: "MOVICOL Junior aromafrei", 1 March 2016 (2016-03-01), XP055619487, Retrieved from the Internet <URL:https://www.google.de/url?sa=t&rct=j&q=&esrc=s&source=web&cd=1&ved=2ahUKEwjnlJOVlcPkAhWNFxQKHcdLC2QQFjAAegQIABAC&url=https%3A%2F%2Fwww.norgine.de%2Fwp-content%2Fuploads%2F2018%2F01%2FGebrauchsanweisung-Movicol-Junior-aromafrei-26.04.2017.pdf&usg=AOvVaw3aVrhv2BOyUkViKxpFpwn8> [retrieved on 20190906]

## Description

### [Technical Field]

The present invention relates to a pharmaceutical composition for treating chronic constipation.

### [Background]

Constipation is a disorder accompanied with unpleasant symptoms such as dyschesia, feeling of incomplete evacuation, and abdominal pain due to decrease of stool frequency, decrease of quantity per bowel movement, and hardening of stool. Treatment has been conducted focusing on the improvement in diet and lifestyle, self-medication by commercial products, and drugs in a medical institution as countermeasures for constipation.

MOVICOL (Norgine Co., Ltd) has been known as a therapeutic drug for constipation. MOVICOL includes macrogol 3350 (European pharmacopoeia), sodium chloride, sodium bicarbonate, and potassium chloride and elicits an effect by increasing the stool volume through accumulating water in the colon and retaining the water of the colon. The administration period of MOVICOL is usually considered to be two weeks. Document CINCA R ET AL: "Randomised clinical trial: macrogol/PEG 3350+electrolytes versus prucalopride in the treatment of chronic constipation - a comparison in a controlled environment", ALIMENTARY PHARMACOLOGY & THERAPEUTICS, BLACKWELL SCIENTIFIC PUBLICATIONS LTD., CAMBRIDGE, GB, vol. 37, no. 9, 11 March 2013 (2013-03-11), pages 876-886, DOI: 10.1111/APT.12278 discloses a study treatment with two sachets Movicol (PEG 3350+E, which means PEG3350 plus electrolytes, 13.13g), administered in a dosage adapted according to the aspect of the stool calculated based on the Bristol stool Chart: see page 878, left column, "study treatment".

### [Summary of Invention]

### [Technical Problem]

The present invention is concerned with providing a pharmaceutical composition that can be used to treat chronic constipation even in long-term administration.

### [Means for Solving the Invention]

As a result of the investigation conducted by the inventors, they have found that using a pharmaceutical composition including polyethylene glycol, sodium chloride, sodium bicarbonate, and potassium chloride can show effectiveness for chronic constipation even in extremely long-term administration, and they have devised the present invention.

The present invention consists of the following embodiments.
(1) In the first embodiment of the invention, there is provided a pharmaceutical composition for use in the treatment of chronic constipation characterized by containing polyethylene glycol, sodium chloride, sodium bicarbonate, and potassium chloride, wherein the composition is administered according to the following dosage adjustment regimen, wherein stool is assessed using the Bristol Stool Scale:
a) a starting dose of 2 sachets once a day;
b) increasing or decreasing the dose by 2 sachets per day, after assessment of the stool;
   (i) wherein when the stool on the Scale is 1 or 2, or no defecation has occurred in a day then the dose is increased, every other day, up to a maximum of 6 sachets per day,
      - wherein if 4 sachets the dose is 2 sachets twice a day (morning and evening); or
      - wherein if 6 sachets the dose is administered as 2 sachets x 1 time and 4 sachets x 1 time (morning and evening);
   (ii) wherein when the stool on the Scale is 3, 4 or 5, the dose is not changed; and
   (iii) wherein when the stool on the Scale is 6 or 7, the dose is reduced by 2 sachets or dosing is discontinued.
   (iv) wherein following discontinuation of the administration, if the stool on the Scale is 3 or more then the discontinuation is maintained or if the stool on the Scale is 1 or 2 or no defecation for 1 day, resume above administration regimen starting with 2 sachets per day.
wherein the patient is aged 15 years or above, and wherein the composition of the sachet is the following:

| | |
|---|---|
| Macrogol | 6.5625 g |
| Sodium chloride | 0.1754 g |
| Sodium bicarbonate | 0.0893 g |
| Potassium chloride | 0.0251g |

(2) In a second embodiment of the invention there is provided a pharmaceutical composition for use in the treatment of chronic constipation characterized by containing polyethylene glycol, sodium chloride, sodium bicarbonate, and potassium chloride, wherein the composition is administered according to the following dosage adjustment regimen, wherein stool is assessed using the Bristol Stool Scale:
a) a starting dose of 2 sachets once a day;
b) increasing or decreasing the dose by 2 sachets per day, after assessment of the stool;
   i) wherein when the stool on the Scale is 1 or 2, then the dose is increased, every other day, up to a maximum of 6 sachets per day,
      - wherein if 4 sachets the dose is 2 sachets twice a day (morning and evening); or
      - wherein if 6 sachets the dose is administered as 2 sachets x 1 time and 4 sachets x 1 time (morning and evening);
   (ii) wherein when the stool on the Scale is 3, 4 or 5, the dose is not changed; and
   (iii) wherein when the stool on the Scale is 6 or 7, the dose is reduced by 2 sachets or dosing is discontinued;
wherein the patient is aged 12 to 14 years old and wherein the composition of the sachet is the following:

| | |
|---|---|
| Macrogol | 6.5625 g |
| Sodium chloride | 0.1754 g |
| Sodium bicarbonate | 0.0893 g |
| Potassium chloride | 0.0251 g. |

(3) In a third embodiment of the invention there is provided a pharmaceutical composition for use in the treatment of chronic constipation characterized by containing polyethylene glycol, sodium chloride, sodium bicarbonate, and potassium chloride, wherein the composition is administered according to the following dosage adjustment regimen, wherein stool is assessed using the Bristol Stool Scale:
a) a starting dose of 1 sachet once a day (age 2 to 6 years) or 2 sachets per day (age 7 to 11 years);
b) increasing or decreasing the dose by 1 sachet per day, after assessment of the stool;
   i) wherein when the stool on the Scale is 1 or 2, then the dose is increased, every other day, up to a maximum of 4 sachets per day,
      - wherein if 1 sachet, the dose is 1 sachet once a day;]
      - wherein if 2 sachets the dose is 2 sachets once a day
      - wherein if 3 sachets, the dose is 1 sachet x 1 time and 2 sachets x 1 time (morning and evening; or
      - wherein if 4 sachets the dose is 2 sachets twice a day (morning and evening);
   (ii) wherein when the stool on the Scale is 3 or 4, the dose is not changed or increased by 1 sachet if there is pain or anal bleeding during defecation;
   (iii) wherein when the stool on the Scale is 5, the dose is decreased by 1 sachet per day, and
   (iv) wherein when the stool on the Scale is 6 or 7, the dose is reduced by 2 sachets or dosing is discontinued.
wherein the patient is aged between 2 and 11 years, and wherein the composition of the sachet is the following:

| | |
|---|---|
| Macrogol | 6.5625 g |
| Sodium chloride | 0.1754 g |
| Sodium bicarbonate | 0.0893 g |
| Potassium chloride | 0.0251 g. |

(4) A pharmaceutical composition, as defined above, for treating chronic constipation characterized by containing polyethylene glycol, sodium chloride, sodium bicarbonate, and potassium chloride and used to treat patients with chronic constipation over a long period of treatment including once or multiple administration periods.
(5) The pharmaceutical composition according to any of (1) to (4), wherein the dosage is increased when the stool is separate hard lumps (stage 1), or is sausage-shaped, but lumpy (stage 2)
(6) The pharmaceutical composition according to any of (1) to (5), wherein the dosage is decreased when the stool is a mushy stool (stage 6), or watery stool (stage 7).
(7) The treatment period includes one or more discontinuation periods.
The pharmaceutical composition according to any of (1) to (6), wherein each discontinuation period is set during each administration period.
(8) The pharmaceutical composition according to (7), wherein the discontinuation period is started when the stool is a mushy stool (stage 6) or watery stool (stage 7).
(9) The pharmaceutical composition according to (8), wherein the discontinuation period is ended when the stool is separate hard lumps (stage 1), or sausage-shaped, but lumpy (stage 2), or when there is no daily bowel movement.
(10) The pharmaceutical composition according to (1) wherein the chronic constipation is functional chronic constipation.
(11) The pharmaceutical composition according to (1), wherein the chronic constipation is not symptomatic chronic constipation, chemical chronic constipation, constipation-predominant irritable bowel syndrome, or organic chronic constipation.
(12) The pharmaceutical composition according to (11), wherein the symptomatic chronic constipation is caused by Parkinson's disease or multiple sclerosis.
(7) The pharmaceutical composition according to any of (1) to (9), wherein the chronic constipation is not constipation caused by organic disease.

### [Effect of the Invention]

According to the present invention, a pharmaceutical composition is provided that can be used to treat chronic constipation even in long-term administration. Moreover, a pharmaceutical composition is provided which is used to treat chronic constipation that can be stopped or prevented from reoccurring depending on the patient's symptoms.

### [Brief Description of Drawings]

[Fig. 1]

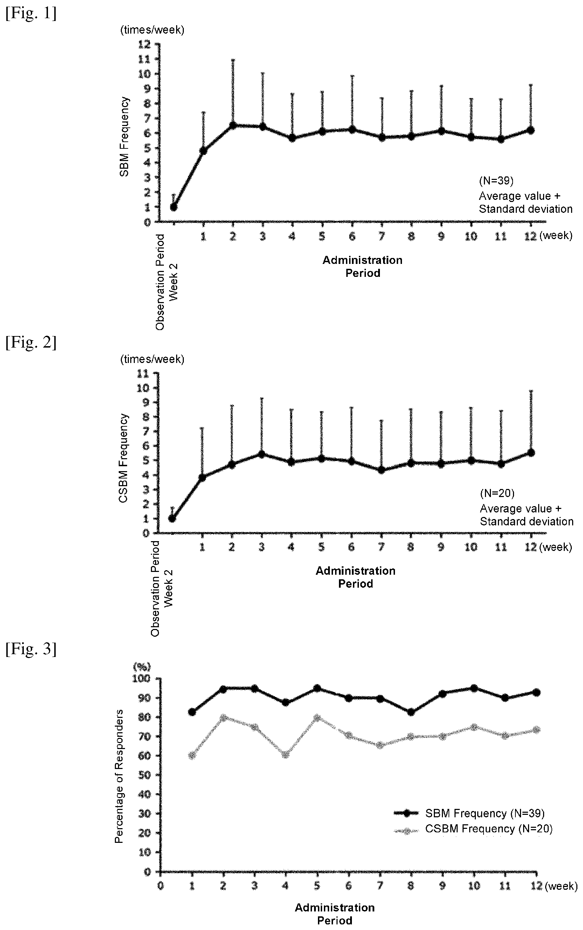

   Fig. 1 shows the transition of spontaneous bowel movement frequency.
[Fig. 2]
   Fig. 2 shows the transition of complete spontaneous bowel movement frequency.
[Fig. 3]
   Fig. 3 shows the transition of percentage of the responder in spontaneous stool frequency and complete spontaneous bowel movement frequency.
[Fig. 4]

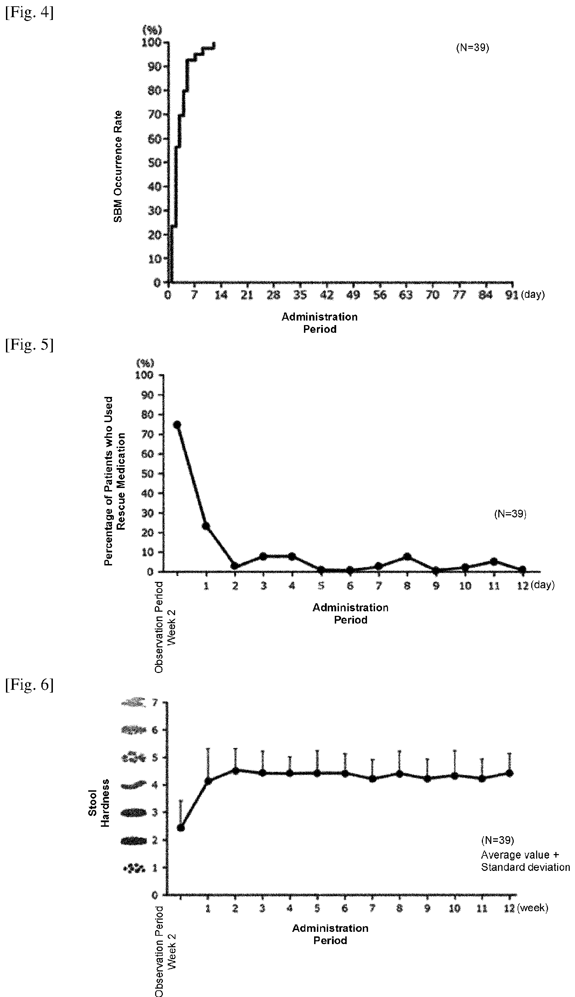

   Fig. 4 shows the number of days until the first manifestation of spontaneous bowel movement.
[Fig. 5]
   Fig. 5 shows the transition of percentage of patients using relief drugs.
[Fig. 6]
   Fig. 6 shows the transition of the median of stool hardness based on the Bristol Stool Scale.
[Fig. 7]

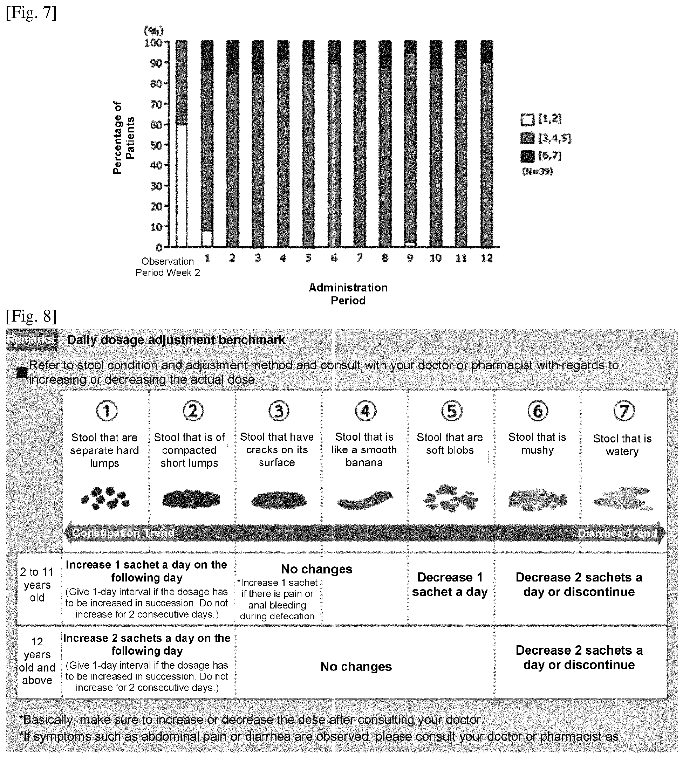

   Fig. 7 shows the percentage when the stool hardness scale is divided into 3 classifications based on the Bristol Stool Scale.
[Fig. 8]
   Fig. 8 shows the illustration describing the relationship with the condition of stool and the method of administration.

### [Mode for Implementing the Invention]

The first embodiment of the present invention relates to a pharmaceutical composition for treating chronic constipation containing polyethylene glycol, sodium chloride, sodium bicarbonate, and potassium chloride and is used to treat patients with chronic constipation over a long period of treatment including one or multiple administration periods.

So far, polyethylene glycol preparations have been used as a therapeutic drug for chronic constipation, but the administration period was basically two weeks. Consulting a doctor in long-term dosing has not been required and no specific examinations have been performed related to long-term dose. Note that long-term dose generally means taking for a long time continuously every day, and the setting of discontinuation period, resuming the treatment period after the discontinuation period, and also the obtaining of an equivalent effect have not been thought about at all until now.

Even if the pharmaceutical composition according to this embodiment is given for a long time (while repeating discontinuation and resumption), its effect is not attenuated. In addition, high safety is secured for long-term dosage. Furthermore, even if a discontinuation period is set during the treatment period, the adverse effects do not occur because of this, and the effect and safety can be maintained by restarting administration the same as before discontinuation.

The polyethylene glycol contained in the pharmaceutical composition according to the present embodiment is preferably macrogol, and more preferably macrogol 3350 (European Pharmacopoeia) or macrogol 4000 (Japanese Pharmacopoeia). But these can be changed and can be used because there is no basic difference between macrogol 3350 (European Pharmacopoeia) and macrogol 4000 (Japanese Pharmacopoeia).

It is preferable that the pharmaceutical composition according to the present embodiment be wrapped individually.

It is preferable that the pharmaceutical composition according to the present embodiment be dissolved in water before administering. The quantity of water to dissolve one sachet of the pharmaceutical composition is preferably 20-150 ml, more preferably 40-100 ml, and even more preferably 60-70 ml.

The pharmaceutical composition according to the present embodiment shows an effect depending on the quantity and not on the density of polyethylene glycol to be administered. Therefore, as for dosing method of the pharmaceutical composition according to the present embodiment can be indicated with, for example, "Administer by dissolving in approximately 1/3 cup of water (approximately 60 ml) per 1 sachet. Moreover, administer 2 sachets by dissolving in approximately 120 ml of water, and 180 ml of water in the case of 3 sachets." or "If administration is difficult due to taste, it can be administered by dissolving it in other drinks like apple juice or cold drinks." In addition, it is preferably indicated with "This medicine is administered by dissolving it in water but, the water at this time will become a stool without mostly being absorbed by the body. Therefore, it is vital to drink an adequate quantity of water other than this solution."

"Treatment" in the present specification means to suppress the symptoms of chronic constipation and/or to cure chronic constipation.

The pharmaceutical composition according to the present embodiment is used in treating patients with chronic constipation for over a long treatment period. "Treatment period" in the present specification is the period where the administration period is combined with discontinuation periods set as needed. When setting of a discontinuation period is not required, the treatment period corresponds to the administration period. The "Administration Period" in the present specification is the period when the pharmaceutical composition according to the present embodiment is administered. The "Discontinuation Period" in the present specification is the period when the pharmaceutical composition of this present embodiment is not administered.

The administration period includes one or multiple administration periods. On the other hand, the discontinuation period includes one or multiple discontinuation periods. It is preferable that the discontinuation period be set between each administration period. For example, when a discontinuation period is set once, it is preferable that the treatment period sequentially contain a first administration period, first discontinuation period, and second administration period. If two discontinuation periods are set, the treatment period shall preferably sequentially contain the first administration period, first discontinuation period, second administration period, second discontinuation period, and third discontinuation period.

The effect of the pharmaceutical composition according to the present embodiment is not attenuated even over a long administration period. In other words, a stable effect is shown over the long term. In addition, high safety is secured even if the administration period extends over the long term.

The pharmaceutical composition according to the embodiment can be administered until the chronic constipation is cured. Although there is no upper limit to the administration period, the upper limit may be set, for example, to 50 years, 40 years, 30 years, 20 years, 10 years, 8 years, 6 years, 4 years, 2 years, and 1 year because it is generally difficult to completely cure chronic constipation in adults.

It is preferable to adjust the dosage of the pharmaceutical composition according to the present embodiment depending on the state of the stool classified in 7 stages as shown below.
Stage 1: Separate hard lumps
Stage 2: Sausage-shaped, but lumpy
Stage 3: Cracks on its surface
Stage 4: Smooth and banana-shaped
Stage 5: Soft semi-solid
Stage 6: Mushy stool
Stage 7: Watery stool

The dosage of the pharmaceutical composition according to the present embodiment is preferably increased in the case of stage 1 or stage 2 mentioned above. Preferably, the dosage of the pharmaceutical composition according to the present embodiment is reduced in the case of stage 6 or 7.

The discontinuation period is set appropriately depending on the patient's symptoms and the number of days are stipulated appropriately. No adverse effect occurs because of this even if the discontinuation period is set during the treatment period, and the effect and safety last by restarting administration the same as before discontinuation. It is preferable that the discontinuation period start when the stage is at stage 6 or 7. It is preferable to end the discontinuation period when the stage is at stage 1 or 2. In addition, it is preferable that the discontinuation period end when there's no daily bowel movement.

The dosage of the pharmaceutical composition according to the present embodiment can be adjusted depending on the Bristol stool scale. Preferably the dosage of the pharmaceutical composition according to the present embodiment is increased when the Bristol stool scale is 1 or 2, and preferably the dosage of the pharmaceutical composition according to the present embodiment is reduced when the Bristol stool scale is 5-7 (particularly, if 6 or 7).

It is preferable to start the discontinuation period when the Bristol stool scale is 6 or 7. Preferably, the discontinuation period is ended when the Bristol stool scale is 1 or 2. In addition, it is preferable to end the discontinuation period when there is no daily bowel movement.

The Bristol stool scale is an index to prescribe the state of the stool by its shape and hardness, and it is stipulated as follows.
Scale 1: Separate hard lumps, like nuts (hard to pass)
Scale 2: Sausage-shaped but lumpy (massive)
Scale 3: Sausage-shaped but with cracks on the surface ____
Scale 4: Sausage or snake-like, smooth and soft
Scale 5: Soft blobs with clear-cut edges (easy to pass)
Scale 6: Fluffy pieces with ragged edges, mushy stool
Scale 7: Watery, no solid pieces

For example, functional chronic constipation, symptomatic chronic constipation, chemical chronic constipation, and organic chronic constipation are cited as chronic constipation. Functional chronic constipation may be the subject of the pharmaceutical composition according to the present embodiment. Symptomatic chronic constipation, constipation-predominant irritable bowel syndrome, chemical chronic constipation, and organic constipation may be excluded as subjects of the pharmaceutical composition according to the present embodiment. For example, chronic constipation caused by Parkinson's disease and multiple sclerosis are cited as symptomatic chronic constipation. While on the other hand, chronic constipation caused by opioid and psychotropic drugs are cited as chemical constipation. Constipation caused by organic disease may also be excluded as subjects of the pharmaceutical composition according to the present embodiment.

The patient is preferably human. For example, patients ages 2-6 years old, 7-11 years old, 12 years and above, or 15 years and above. The human patients may be male or female.

The pharmaceutical composition according to the present invention is preferably taken orally.

In order to understand the usage of the pharmaceutical composition according to the present invention, it is convenient to have an illustration listing the relationship of the stool condition to the administration method. For example, the illustration mentioned in Fig. 8 classifies the condition of the stool into 7 stages with letters and drawings, and lists the administration methods at every stage. Such an illustration is convenient when the patient themselves take the medication and when the doctor is giving instruction. The illustration can be included in various things (for example, booklets, deskpads).

### [Examples]

Hereinafter, the present invention will be described in more detail by using examples.

### <Therapeutic Drug>

A therapeutic drug containing the following ingredients was prepared in one sachet.
Macrogol 3350 (European Pharmacopoeia) (6.5625 g)
Sodium chloride (0.1754 g)
Sodium bicarbonate (0.0893 g)
Potassium chloride (0.0251 g)

### Example 1

### <Subjects>

153 patients with chronic constipation (15 years old and above).

About 85% of patients suffered from chronic functional constipation, and the rest suffered from constipation-predominant irritable bowel syndrome.

### <Dosage and Administration>

Starting with an administration of 2 sachets a day, depending on the condition of the subject, the dosage was adjusted based on the following Dosage Adjustment Standard. Dosage increases and decreases were carried out in units of 2 sachets, and the upper limit of dosing was set to 6 sachets per day. The timing of administration was once a day or twice a day according to the dosage below, and in the case of twice a day administration, it was in the morning and evening.
If 2 sachets: 2 sachets once a day
If 4 sachets: 2 sachets twice a day (morning and evening)
If 6 sachets: (2 sachets × 1 time, 4 sachets × 1 time) a day (morning and evening)

### <Dosage Adjustment Standard>

If the Bristol stool scale is 1 or 2, or if there is no defecation in a day, increase the dosage every other day until it reaches a Bristol stool scale of 3 or 4.

If the Bristol stool scale is 3, 4 or 5, do not change the dosage.

If the Bristol stool scale is 6 or 7, reduce dose by 2 sachets or discontinue the administration. If there is no defecation for 1 day, or even if there was, if the Bristol stool scale is 1 or 2 after the discontinuation of administration, resume from 2 sachets once a day as starting dosage. If the Bristol stool shape scale is 3 or more, continue the suspension of the administration.

### <Clinical Trial Procedure (Drug discontinuation standard)>

If the frequency of complete spontaneous bowel movement (CSBM) 2 weeks prior to the hospital visit is 6 or more, reduce the dosage by 2 sachets or discontinue the administration. If the frequency of spontaneous bowel movement (SBM) in one week becomes less than three times after the suspension of administration, resume the administration at the dose before the suspension.

### <Results>

The results of the long-term administration of the therapeutic drug are shown on the table below. "Spontaneous bowel movement (SBM)" is defecation not induced by laxatives or due to enema or stool extraction. "Complete spontaneous bowel movement (CSBM)" refers to spontaneous bowel movement without the sensation of incomplete evacuation. "Responder" means a subject whose defecation frequency per week improved to more than once since before the drug administration, and defecates three times or more. "Rescue medication" means bisacodyl suppository (10 mg), and it is used when defecation is not observed for more than 72 consecutive hours.

As shown in Table 1, a stable SBM frequency was confirmed from Week 2 to Week 52.

### (Table 1)

**Table 1**

| Treatment Period | No. of Subjects (n) | SBM Frequency (Average value ±Standard deviation) |
|---|---|---|
| Week 1 | 153 | 4.50±2.37 |
| Week 2 | 153 | 5.86±2.88 |
| Week 4 | 150 | 5.45±2.78 |
| Week 12 | 147 | 6.04±2.48 |
| Week 24 | 138 | 5.99±2.82 |
| Week 36 | 132 | 6.05±3.45 |
| Week 48 | 130 | 5.92±2.60 |
| Week 52 | 81 | 5.78±2.42 |

As shown in Table 2, a stable total bowel movement frequency was confirmed from Week 2 to Week 52.

### (Table 2)

**Table 2**

| Treatment Period | No. of Subjects (n) | Total Bowel Movement Frequency (Average value ± Standard deviation) |
|---|---|---|
| Week 1 | 153 | 4.58±2.31 |
| Week 2 | 153 | 5.90±2.83 |
| Week 4 | 150 | 5.48±2.74 |
| Week 12 | 147 | 6.05±2.48 |
| Week 24 | 138 | 6.00±2.81 |
| Week 36 | 132 | 6.06±3.45 |
| Week 48 | 130 | 5.92±2.60 |
| Week 52 | 81 | 5.83±2.46 |

As shown in Table 3, it was confirmed that the proportion of SBM frequency of responders was kept high from Week 1 to Week 52.

### (Table 3)

**Table 3**

| Treatment Period | Percentage of SBM Frequency of Responders |
|---|---|
| Week 1 | 78.4% |
| Week 2 | 88.9% |
| Week 4 | 81.3% |
| Week 12 | 91.2% |
| Week 24 | 88.4% |
| Week 36 | 92.4% |
| Week 48 | 90.8% |
| Week 52 | 88.9% |

As shown in Table 4, it was confirmed that the usage rate of rescue medication was kept low from Week 2 to Week 52.

### (Table 4)

**Table 4**

| Treatment Period | Usage Status of Rescue Medication |
|---|---|
| Week 1 | 6.5% |
| Week 2 | 2.6% |
| Week 4 | 3.3% |
| Week 12 | 0.7% |
| Week 24 | 0.7% |
| Week 36 | 0.8% |
| Week 48 | 0.8% |
| Week 52 | 0.8% |

As shown in Tables 5 and 6, it was confirmed that the improved stool hardness was stably maintained from Week 1 to Week 52.

### (Table 5)

**Table 5**

| Treatment Period | No. of Subjects (n) | Bristol Stool Scale (Median ± Standard deviation) |
|---|---|---|
| Week 1 | 148 | 3.93±1.25 |
| Week 2 | 152 | 4.36±1.06 |
| Week 4 | 150 | 4.11±1.08 |
| Week 12 | 147 | 4.12±0.95 |
| Week 24 | 137 | 3.94±1.03 |
| Week 36 | 131 | 4.07±0.81 |
| Week 48 | 130 | 4.07±0.89 |
| Week 52 | 81 | 4.11±0.91 |

### (Table 6)

**Table 6**

| Treatment Period | No. of Subjects (n) | Bristol Stool Scale (Average value ± Standard deviation) |
|---|---|---|
| Week 1 | 148 | 3.93±1.07 |
| Week 2 | 152 | 4.40±0.94 |
| Week 4 | 150 | 4.23±0.98 |
| Week 12 | 147 | 4.24±0.82 |
| Week 24 | 137 | 4.00±0.93 |
| Week 36 | 131 | 4.11±0.74 |
| Week 48 | 130 | 4.11±0.81 |
| Week 52 | 81 | 4.15±0.81 |

As shown in Table 7, it was confirmed that the number of administered sachets of the therapeutic drug was stable from Week 1 to Week 52. Furthermore, the number of administered sachets at Week 52 is few compared to the other weeks, but this is due to the non-inclusion of the number post-hospital visit sachets with regard to subjects who came to the hospital before day 7 of week 52.

### (Table 7)

**Table 7**

| Treatment Period | No. of Subjects (n) | No. of Administered Therapeutic Drug Sachets (Average value ± Standard deviation) |
|---|---|---|
| Week 1 | 153 | 21.5±7.3 sachets |
| Week 2 | 153 | 24.3±12.1 sachets |
| Week 4 | 152 | 21.7±13.4 sachets |
| Week 12 | 147 | 22.6±13.3 sachets |
| Week 24 | 139 | 22.0±13.9 sachets |
| Week 36 | 133 | 20.9±13.2 sachets |
| Week 48 | 130 | 20.8±13.3 sachets |
| Week 52 | 118 | 15.7±13.4 sachets |

As shown in Table 8, a discontinuation period was set during the treatment period as necessary.

### (Table 8)

**Table 8**

| | No. of Subjects (n) | Discontinuation Period (Average value ± Standard deviation) |
|---|---|---|
| Discontinuation due to improvement in symptoms | 23 | 60.2±84.1 days |
| Discontinuation due to all reasons | 143 | 55.2±80.4 days |

Table 9 shows the No. of sachets used and the SBM frequency at -8 to -14 days (2 weeks before) and -1 to -7 days (1 week before) before the start of discontinuation period, as well as at 1 to 7 days (1 week after), 8 to 14 days (2 weeks after), and 15 to 21 days (3 weeks after) after the end of discontinuation period, and then Table 10 shows the SBM frequency per sachet of the drug. Table 11 shows the percentage of the SBM frequency per 1 sachet of the drug after 1 week, 2 weeks, and 3 weeks with regards to the average value of SBM frequency per 1 sachet of the drug at 1 week and 2 weeks before. If the percentage is close to 1, it indicates that there was no change in efficacy before and after the drug discontinuation period. As shown in Table 11, the percentages after 1 week, 2 weeks, and 3 weeks were 0.95, 1.08, and 1.21, respectively, which were values close to 1.

### (Table 9)

**Table 9**

| | No. of Sachets Used | | | | | SBM Frequency | | | | |
|---|---|---|---|---|---|---|---|---|---|---|
| | Before discontinuation | | After resumption | | | Before discontinuation | | After resumption | | |
| | 2 weeks before | 1 week before | 1 week after | 2 weeks after | 3 weeks after | 2 weeks before | 1 week before | 1 week after | 2 weeks after | 3 weeks after |
| Average value | 14 | 13 | 14 | 13 | 12 | 6 | 6 | 6 | 6 | 6 |
| Maximum value | 42 | 38 | 42 | 42 | 42 | 17 | 17 | 12 | 14 | 15 |
| Minimum value | 2 | 2 | 2 | 2 | 2 | 2 | 1 | 1 | 1 | 1 |

### (Table 10)

**Table 10**

| | SBM Frequency per 1 sachet | | | | |
|---|---|---|---|---|---|
| | Discontinuation Period | Before | After Resumption | | |
| | 2 weeks before | 1 week before | 1 week after | 2 weeks after | 3 weeks after |
| Average value | 0.63 | 0.58 | 0.54 | 0.62 | 0.64 |
| Maximum value | 3.00 | 2.75 | 2.00 | 4.00 | 4.00 |
| Minimum value | 0.07 | 0.10 | 0.07 | 0.13 | 0.14 |

### (Table 11)

**Table 11**

| | SBM Frequency After Resumption / SBM Frequency Before Discontinuation | | |
|---|---|---|---|
| | 1 week after | 2 weeks after | 3 weeks after |
| Average value | 0.95 | 1.08 | 1.21 |
| Maximum value | 4.57 | 5.76 | 6.83 |
| Minimum value | 0.20 | 0.21 | 0.19 |

As shown in Tables 12 to 14, the sodium (Na), potassium (K), and chloride (Cl) in the blood were stable from week 4 to week 52, no symptoms such as dehydration were observed, and it was confirmed that it was excellent in safety. The standard value for sodium concentration in blood is 136 to 147 mEq/L. The standard value for potassium concentration in blood is 3.6 to 5.0 mEq/L. The standard value for chloride concentration in blood is 98 to 109 mEq/L.

**(Table 12)**

| Table 12: Sodium within the blood | | | | | |
|---|---|---|---|---|---|
| Treatment Period | | | Before Treatment | | |
| | | | Low | Within standard | High |
| Week 4 | After Treatment | Low | 0 | 0 | 0 |
| | | Within standard | 0 | 135 | 0 |
| | | High | 0 | 0 | 0 |
| Week 12 | After Treatment | Low | 0 | 1 | 0 |
| | | Within standard | 0 | 145 | 0 |
| | | High | 0 | 0 | 0 |
| Week 24 | After Treatment | Low | 0 | 0 | 0 |
| | | Within standard | 0 | 138 | 0 |
| | | High | 0 | 0 | 0 |
| Week 36 | After Treatment | Low | 0 | 0 | 0 |
| | | Within standard | 0 | 131 | 0 |
| | | High | 0 | 0 | 0 |
| Week 52 | After Treatment | Low | 0 | 1 | 0 |
| | | Within standard | 0 | 126 | 0 |
| | | High | 0 | 0 | 0 |

| | | | | | |
|---|---|---|---|---|---|
| The numbers in the table indicate the number of subjects. | | | | | |

**(Table 13)**

| Table 13: Potassium Concentration in Blood | | | | | |
|---|---|---|---|---|---|
| Treatment Period | | | Before Treatment | | |
| | | | Low | Within standard | High |
| Week 4 | After Treatment | Low | 2 | 1 | 0 |
| | | Within standard | 1 | 123 | 4 |
| | | High | 0 | 2 | 2 |
| Week 12 | After Treatment | Low | 0 | 0 | 0 |
| | | Within standard | 3 | 135 | 5 |
| | | High | 0 | 3 | 0 |
| Week 24 | After Treatment | Low | 2 | 3 | 0 |
| | | Within standard | 1 | 125 | 3 |
| | | High | 0 | 4 | 0 |
| Week 36 | After Treatment | Low | 0 | 3 | 0 |
| | | Within standard | 3 | 119 | 3 |
| | | High | 0 | 3 | 0 |
| Week 52 | After Treatment | Low | 1 | 3 | 0 |
| | | Within standard | 2 | 114 | 2 |
| | | High | 0 | 4 | 1 |

| | | | | | |
|---|---|---|---|---|---|
| The numbers in the table indicate the number of subjects. | | | | | |

**(Table 14)**

| Table 14: Chloride Concentration in Blood | | | | | |
|---|---|---|---|---|---|
| Treatment Period | | | Before Treatment | | |
| | | | Low | Within standard | High |
| Week 4 | After Treatment | Low | 0 | 0 | 0 |
| | | Within standard | 1 | 123 | 2 |
| | | High | 0 | 8 | 1 |
| Week 12 | After Treatment | Low | 0 | 0 | 0 |
| | | Within standard | 1 | 140 | 3 |
| | | High | 0 | 2 | 0 |
| Week 24 | After Treatment | Low | 0 | 0 | 0 |
| | | Within standard | 1 | 134 | 2 |
| | | High | 0 | 1 | 0 |
| Week 36 | After Treatment | Low | 1 | 0 | 0 |
| | | Within standard | 0 | 127 | 2 |
| | | High | 0 | 1 | 0 |
| Week 52 | After Treatment | Low | 0 | 0 | 0 |
| | | Within standard | 1 | 121 | 1 |
| | | High | 0 | 3 | 1 |

| | | | | | |
|---|---|---|---|---|---|
| The numbers in the table indicate the number of subjects. | | | | | |

### Example 2

### <Subjects>

39 patients (2 to 14 years old) with chronic constipation.

### <Dosage and Administration>

### (1) For 2 to 11 years old

The dose at the start was 1 sachet once a day for patients aged 2 to 6, and 2 sachets once a day for patients aged 7 to 11, and then the dose was adjusted according to the condition of the patient. Dose increases were made in units of 1 sachet a day, and the upper limit of dosage was set to 4 sachets per day. The timing of administration was once a day or twice a day according to the dosages below, and in the case of twice a day administration, it was in the morning and evening.
If 1 sachet: 1 sachet once a day
If 2 sachets: 2 sachets once a day
If 3 sachets: (1 sachet × 1 time, 2 sachets × 1 time)/day (morning and evening)
If 4 sachets: 2 sachets twice a day (morning and evening)

### (2) For 12 to 14 years old

Starting with administration of 2 sachets a day, the dosage was adjusted depending on the condition of the patient. Dose increases and decreases were made in units of 2 sachets a day, and the upper limit of dosage was set to 6 sachets per day. The timing of administration was once a day or twice a day according to the dosages below, and in the case of twice a day administration, it was in the morning and evening.
If 2 sachets: 2 sachets once a day
If 4 sachets: 2 sachets twice a day (morning and evening)
If 6 sachets: (2 sachets × 1 time, 4 sachets × 1 time) a day (morning and evening)

### <Results>

Fig. 1 shows the transition of SBM Frequency.

Fig. 2 shows the transition of CSBM frequency.

Fig. 3 shows the transition of percentage of responders in the SBM Frequency and CSBM Frequency.

Fig. 4 shows the number of days until the first manifestation of SBM.

Fig. 5 shows the transition of the percentage of patients using rescue medication.

Fig. 6 shows the transition of the median of stool hardness based on Bristol stool scale.

Fig. 7 shows the percentage if the stool hardness scores are classified into three groups based on the Bristol stool scale.

## Claims

1. A pharmaceutical composition for use in the treatment of chronic constipation **characterized by** containing polyethylene glycol, sodium chloride, sodium bicarbonate, and potassium chloride, wherein the composition is administered according to the following dosage adjustment regimen, wherein stool is assessed using the Bristol Stool Scale:
a) a starting dose of 2 sachets once a day;
b) increasing or decreasing the dose by 2 sachets per day, after assessment of the stool;
(i) wherein when the stool on the Scale is 1 or 2, or no defecation has occurred in a day then the dose is increased, every other day, **up** to a maximum of 6 sachets per day,
• wherein if 4 sachets the dose is 2 sachets twice a day (morning and evening); or
• wherein if 6 sachets the dose is administered as 2 sachets x 1 time and 4 sachets x 1 time (morning and evening);
(ii) wherein when the stool on the Scale is 3, 4 or 5, the dose is not changed; and
(iii) wherein when the stool on the Scale is 6 or 7, the dose is reduced by 2 sachets or dosing is discontinued.
(iv) wherein following discontinuation of the administration, if the stool on the Scale is 3 or more then the discontinuation is maintained or if the stool on the Scale is 1 or 2 or no defecation for 1 day, resume above administration regimen starting with 2 sachets per day.
wherein the patient is aged 15 years or above, and wherein the composition of the sachet is the following:
| | |
|---|---|
| Macrogol | 6.5625 g |
| Sodium chloride | 0.1754 g |
| Sodium bicarbonate | 0.0893 g |
| Potassium chloride | 0.0251g |

2. A pharmaceutical composition for use in the treatment of chronic constipation **characterized by** containing polyethylene glycol, sodium chloride, sodium bicarbonate, and potassium chloride, wherein the composition is administered according to the following dosage adjustment regimen, wherein stool is assessed using the Bristol Stool Scale:
a) a starting dose of 2 sachets once a day;
b) increasing or decreasing the dose by 2 sachets per day, after assessment of the stool;
(i) wherein when the stool on the Scale is 1 or 2, then the dose is increased, every other day, up to a maximum of 6 sachets per day,
• wherein if 4 sachets the dose is 2 sachets twice a day (morning and evening); or
• wherein if 6 sachets the dose is administered as 2 sachets x 1 time and 4 sachets x 1 time (morning and evening);
(ii) wherein when the stool on the Scale is 3, 4 or 5, the dose is not changed; and
(iii) wherein when the stool on the Scale is 6 or 7, the dose is reduced by 2 sachets or dosing is discontinued;
wherein the patient is aged 12 to 14 years old and wherein the composition of the sachet is the following:
| | |
|---|---|
| Macrogol | 6.5625 g |
| Sodium chloride | 0.1754 g |
| Sodium bicarbonate | 0.0893 g |
| Potassium chloride | 0.0251 g. |

3. A pharmaceutical composition for use in the treatment of chronic constipation **characterized by** containing polyethylene glycol, sodium chloride, sodium bicarbonate, and potassium chloride, wherein the composition is administered according to the following dosage adjustment regimen, wherein stool is assessed using the Bristol Stool Scale:
a) a starting dose of 1 sachet once a day (age 2 to 6 years) or 2 sachets per day (age 7 to 11 years);
b) increasing or decreasing the dose by 1 sachet per day, after assessment of the stool;
(i) wherein when the stool on the Scale is 1 or 2, then the dose is increased, every other day, up to a maximum of 4 sachets per day,
• wherein if 1 sachet, the dose is 1 sachet once a day;
• wherein if 2 sachets the dose is 2 sachets once a day
• wherein if 3 sachets, the dose is 1 sachet x 1 time and 2 sachets x 1 time (morning and evening; or
• wherein if 4 sachets the dose is 2 sachets twice a day (morning and evening);
(ii) wherein when the stool on the Scale is 3 or 4, the dose is not changed or increased by 1 sachet if there is pain or anal bleeding during defecation;
(iii) wherein when the stool on the Scale is 5, the dose is decreased by 1 sachet per day, and
(iv) wherein when the stool on the Scale is 6 or 7, the dose is reduced by 2 sachets or dosing is discontinued.
wherein the patient is aged between 2 and 11 years, and wherein the composition of the sachet is the following:
| | |
|---|---|
| Macrogol | 6.5625 g |
| Sodium chloride | 0.1754 g |
| Sodium bicarbonate | 0.0893 g |
| Potassium chloride | 0.0251 g. |

4. The pharmaceutical composition for use according to Claim 3 wherein the patient is aged between 2 and 6 years.

5. The pharmaceutical composition for use according to Claim 3 wherein the patient is aged between 7 and 11 years.

6. The pharmaceutical composition for use according to Claim 1 wherein the chronic constipation is chronic functional constipation.

7. The pharmaceutical composition for use according to Claim 1 wherein the chronic constipation is constipation-predominant irritable bowel syndrome.

## Patentansprüche

1. Eine pharmazeutische Zusammensetzung zur Verwendung bei der Behandlung von chronischer Obstipation, **dadurch gekennzeichnet, dass** sie Polyethylenglycol, Natriumchlorid, Natriumhydrogencarbonat und Kaliumchlorid enthält, wobei die Zusammensetzung gemäß dem folgenden Dosierungsanpassungsschema verabreicht wird, wobei der Stuhl anhand der Bristol-Stuhlformen-Skala bewertet wird:
a) eine Anfangsdosis von 2 Beuteln einmal am Tag;
b) Erhöhung oder Verringerung der Dosis um 2 Beutel pro Tag, nach Bewertung des Stuhls;
(i) wobei wenn der Stuhl 1 oder 2 auf der Skala ist oder innerhalb eines Tages kein Stuhlgang stattgefunden hat, die Dosis erhöht wird, jeden zweiten Tag, bis zu maximal 6 Beutel pro Tag,
• wobei bei 4 Beuteln die Dosis 2 Beutel zweimal am Tag (morgens und abends) beträgt; oder
• wobei bei 6 Beuteln die Dosis als einmal 2 Beutel und einmal 4 Beutel (morgens und abends) verabreicht wird;
(ii) wobei wenn der Stuhl 3, 4 oder 5 auf der Skala ist, die Dosis nicht verändert wird; und
(iii) wobei wenn der Stuhl 6 oder 7 auf der Skala ist, die Dosis um 2 Beutel verringert wird oder die Dosierung beendet wird;
(iv) wobei nach Beendigung der Verabreichung, wenn der Stuhl 3 oder mehr auf der Skala ist, die Beendigung beibehalten wird, oder wenn der Stuhl 1 oder 2 auf der Skala ist oder innerhalb eines Tages kein Stuhlgang stattgefunden hat, das obige Verabreichungsschema wieder aufgenommen wird, wobei mit 2 Beuteln pro Tag begonnen wird;
wobei der Patient 15 Jahre oder älter ist, und wobei die Zusammensetzung des Beutels wie folgt ist:
| | |
|---|---|
| Macrogol | 6,5625 g |
| Natriumchlorid | 0,1754 g |
| Natriumhydrogencarbonat | 0,0893 g |
| Kaliumchlorid | 0,0251 g. |

2. Eine pharmazeutische Zusammensetzung zur Verwendung bei der Behandlung von chronischer Obstipation, **dadurch gekennzeichnet, dass** sie Polyethylenglycol, Natriumchlorid, Natriumhydrogencarbonat und Kaliumchlorid enthält, wobei die Zusammensetzung gemäß dem folgenden Dosierungsanpassungsschema verabreicht wird, wobei der Stuhl anhand der Bristol-Stuhlformen-Skala bewertet wird:
a) eine Anfangsdosis von 2 Beuteln einmal am Tag;
b) Erhöhung oder Verringerung der Dosis um 2 Beutel pro Tag, nach Bewertung des Stuhls;
(i) wobei wenn der Stuhl 1 oder 2 auf der Skala ist, die Dosis erhöht wird, jeden zweiten Tag, bis zu maximal 6 Beutel pro Tag,
• wobei bei 4 Beuteln die Dosis 2 Beutel zweimal am Tag (morgens und abends) beträgt; oder
• wobei bei 6 Beuteln die Dosis als einmal 2 Beutel und einmal 4 Beutel (morgens und abends) verabreicht wird;
(ii) wobei wenn der Stuhl 3, 4 oder 5 auf der Skala ist, die Dosis nicht verändert wird; und
(iii) wobei wenn der Stuhl 6 oder 7 auf der Skala ist, die Dosis um 2 Beutel verringert wird oder die Dosierung beendet wird;
wobei der Patient 12 bis 14 Jahre alt ist, und wobei die Zusammensetzung des Beutels wie folgt ist:
| | |
|---|---|
| Macrogol | 6,5625 g |
| Natriumchlorid | 0,1754 g |
| Natriumhydrogencarbonat | 0,0893 g |
| Kaliumchlorid | 0,0251 g. |

3. Eine pharmazeutische Zusammensetzung zur Verwendung bei der Behandlung von chronischer Obstipation, **dadurch gekennzeichnet, dass** sie Polyethylenglycol, Natriumchlorid, Natriumhydrogencarbonat und Kaliumchlorid enthält, wobei die Zusammensetzung gemäß dem folgenden Dosierungsanpassungsschema verabreicht wird, wobei der Stuhl anhand der Bristol-Stuhlformen-Skala bewertet wird:
a) eine Anfangsdosis von 1 Beutel einmal am Tag (Alter 2 bis 6 Jahre) oder 2 Beuteln pro Tag (Alter 7 bis 11 Jahre);
b) Erhöhung oder Verringerung der Dosis um 1 Beutel pro Tag, nach Bewertung des Stuhls;
(i) wobei wenn der Stuhl 1 oder 2 auf der Skala ist, die Dosis erhöht wird, jeden zweiten Tag, bis zu maximal 4 Beutel pro Tag,
• wobei bei 1 Beutel die Dosis 1 Beutel einmal am Tag beträgt;
• wobei bei 2 Beuteln die Dosis 2 Beutel einmal am Tag beträgt;
• wobei bei 3 Beuteln die Dosis einmal 1 Beutel und einmal 2 Beutel (morgens und abends) beträgt; oder
• wobei bei 4 Beuteln die Dosis 2 Beutel zweimal am Tag (morgens und abends) beträgt;
(ii) wobei wenn der Stuhl 3 oder 4 auf der Skala ist, die Dosis nicht verändert wird oder um 1 Beutel erhöht wird, wenn Schmerzen oder Blutungen im Analbereich während des Stuhlgangs auftreten;
(iii) wobei wenn der Stuhl 5 auf der Skala ist, die Dosis um 1 Beutel pro Tag verringert wird; und
(iii) wobei wenn der Stuhl 6 oder 7 auf der Skala ist, die Dosis um 2 Beutel verringert wird oder die Dosierung beendet wird;
wobei der Patient zwischen 2 und 11 Jahre alt ist, und wobei die Zusammensetzung des Beutels wie folgt ist:
| | |
|---|---|
| Macrogol | 6,5625 g |
| Natriumchlorid | 0,1754 g |
| Natriumhydrogencarbonat | 0,0893 g |
| Kaliumchlorid | 0,0251 g. |

4. Die pharmazeutische Zusammensetzung zur Verwendung gemäß Anspruch 3, wobei der Patient zwischen 2 und 6 Jahre alt ist.

5. Die pharmazeutische Zusammensetzung zur Verwendung gemäß Anspruch 3, wobei der Patient zwischen 7 und 11 Jahre alt ist.

6. Die pharmazeutische Zusammensetzung zur Verwendung gemäß Anspruch 1, wobei die chronische Obstipation chronische funktionelle Obstipation ist.

7. Die pharmazeutische Zusammensetzung zur Verwendung gemäß Anspruch 1, wobei die chronische Obstipation Obstipations-prädominantes Reizdarmsyndrom ist.

## Revendications

1. Composition pharmaceutique destinée à être utilisée dans le traitement de la constipation chronique **caractérisée en ce qu'**elle contient du polyéthylène glycol, du chlorure de sodium, du bicarbonate de sodium et du chlorure de potassium, dans laquelle la composition est administrée selon le schéma d'ajustement posologique suivant, dans laquelle les selles sont évaluées à l'aide de l'échelle de Bristol :
a) une dose initiale de 2 sachets une fois par jour ;
b) augmentation ou diminution de la dose, de 2 sachets par jour, après évaluation des selles ;
(i) dans laquelle, lorsque les selles sur l'échelle sont de type 1 ou 2, ou qu'aucune défécation n'a eu lieu dans une journée, alors la dose est augmentée, tous les deux jours, jusqu'à un maximum de 6 sachets par jour,
• dans laquelle, avec 4 sachets, la dose est de 2 sachets deux fois par jour (matin et soir) ; ou
• dans laquelle, avec 6 sachets, la dose est administrée à raison de 2 sachets x 1 fois et 4 sachets x 1 fois (matin et soir) ;
(ii) dans laquelle, lorsque les selles sur l'échelle sont de type 3, 4 ou 5, la dose n'est pas modifiée ; et
(iii) dans laquelle, lorsque les selles sur l'échelle sont de type 6 ou 7, la dose est réduite de 2 sachets ou l'administration est interrompue ;
(iv) dans laquelle, après l'arrêt de l'administration, si les selles sur l'échelle sont de type 3 ou plus, l'arrêt est maintenu ou, si les selles sur l'échelle sont de type 1 ou 2 ou qu'il n'y a aucune défécation pendant 1 jour, il convient de reprendre le schéma d'administration ci-dessus en commençant par 2 sachets par jour ;
dans laquelle le patient a 15 ans ou plus et dans laquelle la composition du sachet est la suivante :
| | |
|---|---|
| Macrogol | 6,5625 g |
| Chlorure de sodium | 0,1754 g |
| Bicarbonate de sodium | 0,0893 g |
| Chlorure de potassium | 0,0251 g |

2. Composition pharmaceutique destinée à être utilisée dans le traitement de la constipation chronique **caractérisée en ce qu'**elle contient du polyéthylène glycol, du chlorure de sodium, du bicarbonate de sodium et du chlorure de potassium, dans laquelle la composition est administrée selon le schéma d'ajustement posologique suivant, dans laquelle les selles sont évaluées à l'aide de l'échelle de Bristol :
a) une dose initiale de 2 sachets une fois par jour ;
b) augmentation ou diminution de la dose, de 2 sachets par jour, après évaluation des selles ;
(i) dans laquelle, lorsque les selles sur l'échelle sont de type 1 ou 2, alors la dose est augmentée, tous les deux jours, jusqu'à un maximum de 6 sachets par jour,
• dans laquelle, avec 4 sachets, la dose est de 2 sachets deux fois par jour (matin et soir) ; ou
• dans laquelle, avec 6 sachets, la dose est administrée à raison de 2 sachets x 1 fois et 4 sachets x 1 fois (matin et soir) ;
(ii) dans laquelle, lorsque les selles sur l'échelle sont de type 3, 4 ou 5, la dose n'est pas modifiée ; et
(iii) dans laquelle, lorsque les selles sur l'échelle sont de type 6 ou 7, la dose est réduite de 2 sachets ou l'administration est interrompue ;
dans laquelle le patient a de 12 à 14 ans et dans laquelle la composition du sachet est la suivante :
| | |
|---|---|
| Macrogol | 6,5625 g |
| Chlorure de sodium | 0,1754 g |
| Bicarbonate de sodium | 0,0893 g |
| Chlorure de potassium | 0,0251 g. |

3. Composition pharmaceutique destinée à être utilisée dans le traitement de la constipation chronique **caractérisée en ce qu'**elle contient du polyéthylène glycol, du chlorure de sodium, du bicarbonate de sodium et du chlorure de potassium, dans laquelle la composition est administrée selon le schéma d'ajustement posologique suivant, dans laquelle les selles sont évaluées à l'aide de l'échelle de Bristol :
a) une dose initiale de 1 sachet une fois par jour (de 2 à 6 ans) ou de 2 sachets par jour (de 7 à 11 ans) ;
b) augmentation ou diminution de la dose, de 1 sachet par jour, après évaluation des selles ;
(i) dans laquelle, lorsque les selles sur l'échelle sont de type 1 ou 2, alors la dose est augmentée, tous les deux jours, jusqu'à un maximum de 4 sachets par jour,
• dans laquelle, avec 1 sachet, la dose est de 1 sachet une fois par jour ;
• dans laquelle, avec 2 sachets, la dose est de 2 sachets une fois par jour ;
• dans laquelle, avec 3 sachets, la dose est de 1 sachet x 1 fois et 2 sachets x 1 fois (matin et soir) ; ou
• dans laquelle, avec 4 sachets, la dose est de 2 sachets deux fois par jour (matin et soir) ;
(ii) dans laquelle, lorsque les selles sur l'échelle sont de type 3 ou 4, la dose n'est pas modifiée ni augmentée de 1 sachet en cas de douleur ou de saignement anal à la défécation ;
(iii) dans laquelle, lorsque les selles sur l'échelle sont de type 5, la dose est diminuée de 1 sachet par jour, et
(iv) dans laquelle, lorsque les selles sur l'échelle sont de type 6 ou 7, la dose est réduite de 2 sachets ou l'administration est interrompue ;
dans laquelle le patient a entre 2 et 11 ans et dans laquelle la composition du sachet est la suivante :
| | |
|---|---|
| Macrogol | 6,5625 g |
| Chlorure de sodium | 0,1754 g |
| Bicarbonate de sodium | 0,0893 g |
| Chlorure de potassium | 0,0251 g. |

4. Composition pharmaceutique pour une utilisation selon la revendication 3, dans laquelle le patient a entre 2 et 6 ans.

5. Composition pharmaceutique pour une utilisation selon la revendication 3, dans laquelle le patient a entre 7 et 11 ans.

6. Composition pharmaceutique pour une utilisation selon la revendication 1, dans laquelle la constipation chronique est une constipation fonctionnelle chronique.

7. Composition pharmaceutique pour une utilisation selon la revendication 1, dans laquelle la constipation chronique est un syndrome de l'intestin irritable avec constipation prédominante.
